# EUROPEAN PATENT APPLICATION

(11) **EP 1 731 599 A1**
(43) Date of publication of application: **13.12.2006**
(21) Application number: 05727508.3
(22) Date of filing: 31.03.2005
(51) Int. Cl.: C12N 7/01, A61K 35/76, A61P 35/00, C12N 15/869, A61K 48/00

(54) **METHOD OF CONSTRUCTING RECOMBINANT HERPES SIMPLEX VIRUS**

(30) Priority: 31.03.2004 JP 2004105273
(71) Applicant: Todo, Tomoki, Tokyo 1350044 (JP)
(72) Inventor: TODO, Tomoki, Tokyo 1350044 (JP); FUKUHARA, Hiroshi, Tokyo 1050014 (JP)
(74) Representative: Smyth, Gyles Darren
(86) International application number: PCT/JP2005/006396
(87) International publication number: WO 2005/103237

(57) **Abstract**

A method is provided for rapidly and reliably constructing recombinant herpes simplex virus (HSV) capable of expressing a target protein in cancer cells. The method for constructing recombinant HSV as claimed in the present invention comprises a first step of inserting into a herpes simplex virus (HSV) genome, a BAC plasmid, which has a IoxP site and an FRT site and into which has been inserted at least one type of marker gene expression cassette between the IoxP site and the FRP site, a second step of constructing a shuttle vector into which has been respectively inserted at least one type of expression cassette of a gene encoding a target protein, at least one type of marker gene, a IoxP site and an FRP site, and inserting the shuttle vector into the IoxP site of the HSV genome using Cre recombinase, and a third step of co-infecting a host with the HSV genome and a vector capable of expressing Flp recombinase, and excising the region between the FRT sites in the genome to produce a target recombinant HSV.

## Description

### BACKGROUND

The present invention relates to a method for constructing recombinant herpes simplex virus capable of expressing a target protein in a target cell, and to a pharmaceutical composition containing this recombinant herpes simplex virus.

In recent years, attempts have been made to construct viruses which selectively replicate in cancer cells and apply those viruses to cancer treatment by modifying virus genomes using genetic engineering techniques based on findings relating to molecular and cellular mechanisms of viral infections, genetic mechanisms relating to oncogenesis, the microbiological mechanism of cancer cell proliferation and so on.

The concept of applying recombinant viruses to cancer treatment was first advocated by Martuza et al. in 1991 (see, for example, Martuza, R.L. et al.; Science 252: 854-6 (1991)). Since many viruses are themselves pathogenic, direct administration to humans and so forth has detrimental effects on normal cells as well. However, by deleting or mutating specific genes using genetic recombination, viruses can be constructed which are incapable of replicating in normal cells, but which are capable of replicating in actively proliferating tumor cells through compensation of the deleted gene function.

Cancer therapy viruses, which have been genetically modified so as to selectively replicate only in cancer cells, replicate in situ upon infecting cancer cells, and cause destruction of cancer cells of the host during that process. Subsequently, antitumor effects are demonstrated through repetition of replication, cell death and infection. On the other hand, since therapeutic viruses which have infected normal cells do not replicate, they do not cause damage to normal tissue.

Examples of such mutant viruses which have been developed thus far include:
- a mutant virus in which the thymidine kinase (tk) gene has been deleted from the genome of herpes simplex virus type I (HSV-1) (see, for example, the above-mentioned Martuza, R.L. et al.; Science 252: 854-6 (1991));
- HSV-1 in which the γ34.5 gene has been deleted and the ICP6 gene has been inactivated (G207) (see, for example, Chahlavi, A. et al.: Neoplasia 1: 162-169 (1999); Hunter, W.D. et al.: J Virol 73: 6319-6326 (1999); Chahlavi, A. et al.: Gene Ther 6: 1751-1758 (1999); Nakamura, S. et al.: Glia 28: 53-65 (1999); Todo, T. et al.: Hum Gene Ther 10: 2741-2755 (1999); Todo, T. et al.: Hum Gene Ther 10: 2869-2878 (1999); Todo, T et al.: Cancer Gene Ther. 7: 939-946 (2000); Markert, J.M. et al.: Gene Ther. 7: 867-874 (2000); Todo, T. et al.: Mol. Ther. 2: 588-595 (2000); Nakano, K. et al.: Mol. Ther. 3: 431-437 (2001); Varghese, S. et al.: Hum. Gene Ther. 12: 999-1010 (2001); Jorgensen, T.J. et al.: Neoplasia 3: 451-456 (2001); and, Todo, T et al.: Tumor Suppressing Viruses, Genes and Drugs - Innovative Cancer Therapy Approaches, San Diego, Academic Press, 45-75 (2001 )); and
- HSV-1 in which ICP47 gene (also referred to as α47 gene) has been inactivated in addition to γ34.5 gene and ICP6 gene (G47Δ) (see, for example, the above-mentioned US Patent Publication No. 2002/0187163A1 and Todo, T. et al.: Proc. Natl. Acad. Sci. USA 98: 6396-6401 (2001)).

Although these mutant viruses are unable to replicate in normal cells, they retain the ability to replicate in tumor cells. G47Δ in particular has high tumor specificity and safety as a result of mutating three genes, and is extremely useful as a therapeutic virus.

On the other hand, cancer therapy viruses not only demonstrate cytocidal effects, but also have the function of evoking antitumor immunity. The inventors of the present invention demonstrated, through research using mice having normal immune systems, that recombinant HSV-1 administered into a tumor not only shows cytocidal effects by proliferating within the tumor, but also enhances antitumor effects by evoking specific antitumor immunity (see, for example, Todo, T. et al.: Hum Gene Ther 10: 2741-2755 (1999); Todo, T. et al.: Hum Gene Ther 10: 2869-2878 (1999); and, Toda, M. et al.: Hum. Gene Ther. 10: 385-393 (1999)). For example, when G207 was administered into N18 tumor (neuroblastoma) prepared subcutaneously in A/J mice, systemic antitumor immunity was induced accompanying an increase in activity of cytotoxic T lymphocytes (CTL) specific to N18 cells, and distal subcutaneous tumor growth or intracerebral tumor growth was inhibited. Mice treated with G207 therapy acquired a tumor-specific defense immunity, and increases in N18 cell-specific CTL activity were maintained for more than one year. Namely, intratumor administration of cancer therapy HSV-1 acts as a cancer vaccine in situ without requiring identification of tumor antigen, offers a simpler procedure than ex vivo methods requiring culturing of tumor cells and so on, and is extremely useful because it has potential to control metastatic foci through treatment of the primary focus by means of systemic antitumor immunity.

The inventors of the present invention confirmed that antitumor effects are enhanced when a cancer therapy virus is combined with expression of an immunity-stimulating gene using a non-proliferating HSV-1 vector in the form of HSV-1 amplicon. An amplicon expressing secretory T cell co-stimulatory factor B7.1-Ig was prepared using G207 as a helper, and the resulting mixed vector was administered directly into a brain tumor or subcutaneous tumor of Neuro2a (mouse neuroblastoma) having low immunogenicity. As a result, although G207 demonstrated cytocidal effects while replicating within tumor cells, since the amplicon persistently secreted B7.1-Ig to the outside from the infected tumor cells, potent antitumor effects and specific antitumor immunity were able to be evoked (see, for example, Todo, T. et al.: Cancer Res 61: 153-161 (2001)). In addition, enhancement of antitumor effects was also obtained with a combination of an amplicon expressing interleukin (IL)-12 and G207 (see, for example, Toda, M. et al.: J. Immunol 160: 4457-4464 (1998)).

In addition to methods using an amplicon, another method for enhancing the antitumor immunity of viral therapy consists of allowing a virus to function as a proliferation vector by directly incorporating a gene encoding a protein involved in cancer treatment into the genome of therapeutic recombinant HSV-1. If a gene encoding a therapy-associated protein is incorporated in a therapeutic recombinant HSV-1 genome such as G207 or G47Δ instead of combining with an amplicon, in addition to obtaining a gene allocation which has proliferated in the tumor, there is the advantage of being able to obtain a stable and large amount of vector at all times.

Construction of recombinant HSV-1 has been carried out by conventional homologous recombination methods. Homologous recombination refers to recombination in which an exogenous gene is incorporated with high probability at a highly homologous location when incorporating that gene into a genome. Thus, in the case there are a plurality of highly homologous locations in a single genome, it is difficult to control where the exogenous gene will be incorporated. Since the genome of HSV-1 is particularly large, in order to obtain a target genetic recombinant, considerable labor is required, including screening, selecting, purifying, and confirming at the molecular level several ten thousands of candidate virus strains, thus resulting in the construction of a single recombinant HSV-1 normally requiring one to two years.

In contrast, a method using a bacterial artificial chromosome (BAC) has been proposed as a method for efficiently incorporating a cancer therapy gene such as an immunostimulatory gene into the genome of a recombinant cancer therapy virus referred to as MGH-1, which has a similar structure to that of G207 (Saeki, Y et al.: Mol. Ther. 3: S45-46 (2001)). In this method, a therapeutic gene, a IoxP site serving as the target sequence of Cre recombinase, an FRT site serving as the target sequence of Flp recombinase, and a BAC plasmid containing at least one marker gene and so on, are first inserted into the genome of a cancer therapy virus. Next, although a shuttle vector incorporating the target therapeutic gene is inserted into this genome, at that time, the Flp-FRT system is used instead of homologous recombination. As a result, the probability of obtaining the target recombinant is increased. A virus is then produced by transforming the resulting virus genome in Vero cells, and excising unnecessary regions of the genome using the Cre-IoxP system.

However, since the Flp-FRT system is used when inserting the shuttle vector in the method described in Saeki, Y. et al.: Mol. Ther. 3: S45-46 (2001), this reaction must be carried out in Escherichia coli having a plasmid which expresses Flp recombinase. Consequently, although both a plasmid expressing Flp recombinase and a shuttle vector plasmid must be transformed in the E. coli, the transformation rate in E. coli is not high. Since the probability of transforming two types of plasmids is the product of the probabilities of the two independent events, the probability of obtaining E. coli containing both is extremely low. In addition, since the E. coli must also have a virus genome in which the above-mentioned BAC plasmid has been inserted, it is difficult to prepare this type of special E. coli in large volume.

In addition, according to the method described in Saeki, Y. et al: Mol. Ther. 3: S45-46 (2001), although a gene encoding a green fluorescent protein (GFP) remains in the final product in the form of the virus genome, the toxicity thereof is a considerable obstacle to applying this method to human cancer therapy.

Moreover, in this method, a red fluorescent protein (RFP) gene is finally incorporated in the portion which is excised due to the function of the Cre-loxP system, and selection of the final product is confirmed by disappearance of the red fluorescence. However, since the timing of the expression of RFP is delayed and the fluorescence thereof is not sufficiently bright, it cannot be said to be optimum for detection. As a result, there is the possibility of a virus genome, in which unnecessary sequences have not been excised due to the Cre-loxP system not functioning properly, being selected as the target genome. This present an obstacle to applications to humans particularly in the case in which a gene encoding an immunogenic or toxic protein is contained in the sequence scheduled to be excised.

Since the method described in Saeki, Y. et al: Mol. Ther. 3: S45-46 (2001) uses a sequence unique to herpes virus for the promoter of the therapeutic gene, there is also the problem of a high likelihood of the occurrence of unexpected mutations caused by homologous recombination.

### SUMMARY

Therefore, in order to solve the above-mentioned problems, an object of the present invention is to provide a method for rapidly and reliably constructing practical recombinant herpes simplex virus capable of expressing a target protein in cancer cells, and being dramatically advanced with respect to safety, ability of the virus to replicate in cancer cells, and antitumor effects.

As a result of conducting extensive studies in consideration of the above-mentioned problems, the inventors of the present invention found that recombinant HSV capable of expressing a target protein can be obtained much more rapidly than with methods of the prior art by using the Cre-IoxP system when inserting a shuttle vector into the HSV genome. In addition, the inventors of the present invention found that the method as claimed in the present invention can be made to be more useful by designing such that the virus of the final product has an IacZ gene instead of GFP for the marker gene; the disappearance of GFP is used to select and confirm the final product; the sequence not present in the naturally-occurring herpes virus genome is used for the promoter of the therapeutic gene; and a stuffer sequence is incorporated in the region excised by the Flp-FRT system in the final stage.

Moreover, the inventors of the present invention also confirmed that by using a third-generation G47Δ virus genome obtained by further modifying G207, as the basic skeleton, when using the method as claimed in the present invention, a practical recombinant herpes simplex virus is obtained which is extremely superior in terms of safety, ability of the virus to replicate in cancer cells and antitumor effects, thereby leading to completion of the present invention.

Namely, the present invention relates to: [1] a method for constructing recombinant herpes simplex virus capable of expressing a target protein in cancer cells, which comprises the steps of: inserting into a herpes simplex virus genome, a BAC plasmid, which has a IoxP site and an FRT site and into which has been inserted at least one type of marker gene expression cassette having a structure in which a marker gene is functionally linked downstream from a promoter, between the IoxP site and the FRP site; constructing a shuttle vector into which has been respectively inserted at least one type of expression cassette of a gene encoding a target protein having a structure in which the gene encoding the target protein is functionally linked downstream from a promoter, at least one type of marker gene, a IoxP site and an FRP site, and inserting the shuttle vector into the IoxP site of the herpes simplex virus genome using Cre recombinase so as to realize a constitution which allows expression of the gene encoding the target protein and the marker gene; and, co-infecting a host with the herpes simplex virus genome obtained in the second step and a vector capable of expressing Flp recombinase, and excising the region between the FRT sites in the genome to produce a target recombinant herpes simplex virus; [2] the method described in [1] above wherein the second step is carried out in a liquid phase; [3] the method described in [1] or [2] wherein a γ34.5 gene and ICP6 gene of the herpes simplex virus are deleted or inactivated prior to the first step; [4] the method described in [3] above wherein ICP47 gene of the herpes simplex virus is additionally deleted or inactivated: [5] the method described in any one of [1] to [4] above wherein the marker gene inserted into the BAC plasmid is a gene encoding green fluorescent protein (GFP) and/or an antibiotic resistance gene; [6] the method described in any one of [1] to [5] above wherein a promoter contained in at least one type of expression cassette of a gene encoding a target protein is a promoter comprising a nucleotide sequence not present in the naturally-occurring herpes simplex virus genome; [7] the method described in any one of [1] to [6] above wherein the promoter comprising a nucleotide sequence not present in the herpes simplex virus genome is CMV promoter; [8] the method described in any one of [1] to [7] wherein the marker gene inserted into the shuttle vector is IacZ gene and/or an antibiotic resistance gene; [9] the method described in [8] above wherein the marker gene inserted into the shuttle vector is an antibiotic resistance gene different from the antibiotic resistance gene inserted into the BAC plasmid; [10] the method described in any one of [1] to [9] above wherein the gene encoding the target protein is one or more genes selected from the group comprising an immunostimulatory gene, a gene encoding a protein having anti-neovascularizing action, a gene encoding a cell membrane fusion protein, and a tumor suppressor gene; [11] the method described in [10] above wherein the immunostimulatory gene is a gene encoding one or more proteins selected from the group comprising co-stimulatory factor, IL-12, IL-18, IL-23, IL-27 and transporter associated with antigen processing (TAP); [12] the method described in [10] above wherein the gene encoding a protein having anti-neovascularizing action is a gene encoding one or more proteins selected from the group comprising endostatin, angiostatin, dominant negative FGF receptor and platelet factor 4; [13] the method described in [10] above wherein the gene encoding a cell membrane fusion protein is a virus surface protein; [14] the method described in [10] above wherein the tumor suppressor gene is p53 gene; [15] the method described in any one of [1] to [14] above wherein the shuttle vector contains a stuffer sequence; [16] the method described in [15] above wherein the stuffer sequence is about 5000 nucleotides or more in length; [17] a recombinant herpes simplex virus constructed according to the method described in any one of [1] to [16] above; [18] a pharmaceutical composition containing a recombinant herpes simplex virus constructed with the method described in any one of [1] to [17] above; [19] the pharmaceutical composition described in [18] above which is a therapeutic or preventive of various cancer diseases; and, [20] a method for preventing or treating cancer comprising administration of the pharmaceutical composition described in [18] above.

According to the present invention, a target recombinant HSV can be obtained in a short period of time and at high yield. According to the present method, construction of a recombinant virus which had previously taken one to two years can now be carried out in two to three months, and what is more, four to five types of different recombinant HSV can be constructed simultaneously.

In addition, the effect of the present invention is further enhanced by using the G47Δ HSV-1 genome as the skeleton, allowing the obtaining of a practical cancer therapy HSV having both high levels of safety and antitumor effects.

Moreover, according to the present invention, effects which evoke antitumor immunity in situ, tumor suppressor effects and anti-neovascularization effects can be imparted to recombinant HSV demonstrating cytocidal effects in tumor cells, thereby making it possible to enhance the antitumor effects of viral therapy.

### DESCRIPTION OF DRAWINGS

FIG. 1 is an explanatory drawing showing an overview of the method for constructing recombinant HSV-1 as claimed in the present invention;
FIG. 2 shows a BAC plasmid used to construct recombinant HSV-1 as claimed in the present invention;
FIG. 3 shows a shuttle vector used in the present invention;
FIG. 4 shows a shuttle vector incorporating IL-18 gene;
FIG. 5 shows a shuttle vector incorporating B7-1-Ig gene;
FIG. 6 shows a shuttle vector incorporating IL-18 gene and B7-1-Ig gene;
FIG. 7 shows the results of an experiment on cytocidal effects against Neuro2a;
FIG. 8 shows the results of an experiment on cytocidal effects against TRAMP;
FIG. 9 shows the results of an experiment on antitumor effects against a G47Δ mouse tumor model expressing IL-18 and/or B7; and

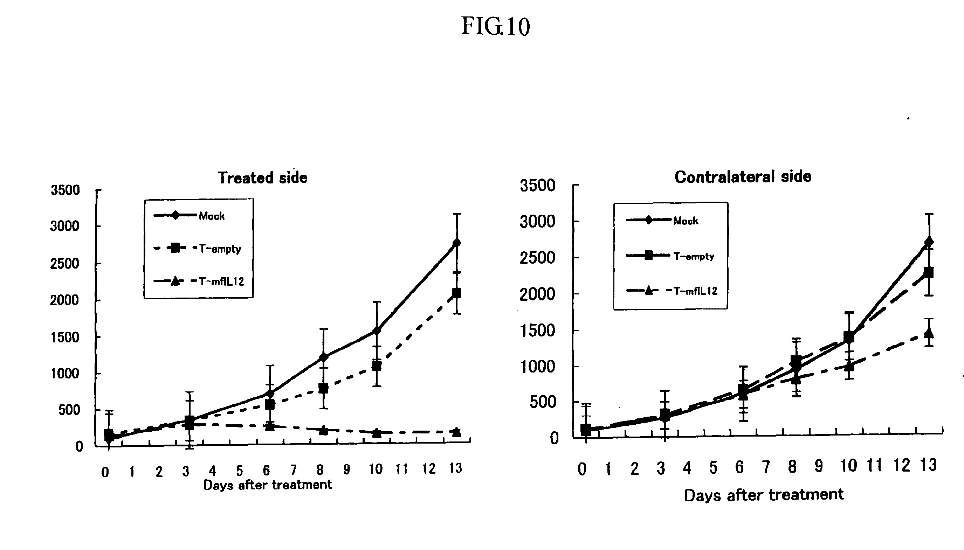

FIG. 10 shows the results of an experiment on antitumor effects against a G47Δ mouse tumor model expressing IL-12.

### DETAILED DESCRIPTION

The following provides a detailed explanation of the present invention by clarifying the significance of the terms and so on used in the present invention.

In the method for constructing recombinant HSV as claimed in the present invention, a IoxP site and an FRT site are first inserted into an HSV genome using a BAC system. On the other hand, a gene encoding a target protein desired to be expressed in cancer cells is inserted into a shuttle vector together with a functionally linked promoter. As a result of inserting and IoxP site and FRP site at suitable locations in this shuttle vector, an HSV genome having a target mutation can be obtained by first inserting the shuttle vector into the HSV genome using Cre recombinase, and then excising the unnecessary portion from the HSV genome using Flp recombinase.

Since a Cre-IoxP system is used in the second step in which the shuttle vector is incorporated in the HSV genome, and a Flp-FRT system is used in the third step in which an unnecessary region is excised, the method can be carried out through the second step in solution in, for example, a test tube or Eppendorf tube, thereby enabling recombination to be carried out rapidly and with high accuracy by a simple procedure. In addition, since it is no longer necessary to insert the shuttle vector into host cells in the second step, the yield can be increased considerably.

In the present invention, there are no particular limitations on the "recombinant herpes simplex virus" provided it is a herpes simplex virus (HSV) which has been altered by gene recombination technology to be able to express a target protein in cancer cells, and may be inserted with any altered or exogenous naturally-occurring HSV gene. In addition, although the HSV type may be type I (HSV-1) or type II (HSV-II), HSV-1 is used preferably.

HSV-1 is classified as an enveloped, double-stranded DNA virus, and has the following characteristics advantageous to cancer therapy: 1) able to infect all types of human cells; 2) virus life cycle and genome sequence have been elucidated; 3) the majority of the functions of the viral genes have been determined, and those genes can be manipulated; and, 4) large genes and multiple genes can be incorporated due to the large size of the virus genome (approx. 152 kb). Moreover, HSV-1 also has the following advantages suitable for clinical application: 5) capable of destroying all cells at a comparatively low multiplicity of infection (MOl); 6) anti-viral drugs exist which inhibit viral replication; 7) anti-HSV-1 antibody in the blood does not affect the spread of infection into cells from virus cells; 8) pre-clinical evaluations of safety and efficacy can be carried out in animals since mice and monkeys exist which are sensitive to HSV-1; and 9) viral DNA stays outside the chromosomes without being incorporated into the genome of host cells.

HSV used in the present invention is preferably HSV which has been altered by genetic recombination technology or has mutated spontaneously so as to be able to proliferate only in cancer cells without proliferating in normal cells prior to the first step (to be simply referred to as "recombinant HSV"). In comparison with the case of using a non-proliferating amplicon, recombinant HSV is able to express more target protein since it proliferates in cancer cells.

Examples of such viruses include HSV in which γ34.5 gene and ICP6 gene have been deleted or inactivated, and HSV in which ICP47 gene has been deleted or inactivated in addition to these two genes.

The γ34.5 gene product is a protein which antagonizes the function of double-stranded RNA-activated protein kinase (PKR). In normal cells, PKR is phosphorylated in response to HSV-1 infection, and this phosphorylates translation initiation factor eIF-2α, resulting in inhibition of viral protein synthesis. Thus, virus replication is inhibited in normal cells if γ34.5 gene does not function. However, in cancer cells, and particularly in cells in which the Ras signal transduction pathway has been activated, since PKR is already inhibited, virus replication is possible even in mutant HSV-1 in which γ34.5 has been deleted.

ICP6 gene is a gene which encodes a large subunit of ribonucleotide reductase (RR). If the RR gene is removed or inactivated, HSV-1 cannot replicate in non-dividing cells (normal cells). However, replication is possible in actively dividing cells in which RR activity is increased by compensating for the missing virus enzyme.

ICP47 protein decreases the expression of MHC class I of infected cells by inhibiting transporter associated with antigen processing (TAP), and acts so as to allow the virus to escape the host's immunosurveillance. Consequently, antitumor immunity is enhanced if ICP47 gene is inactivated since the expression of MHC class I is maintained in infected cancer cells.

Examples of HSV in which γ34.5 gene and ICP6 gene have been deleted or inactivated include the previously described G207 and MGH-1, which has a structure similar to G207, while an example of HSV in which the three γ34.5, ICP6 and ICP47 genes have been deleted or inactivated is the previously described G47Δ. Among these, G47Δ is a triple mutant, which has high levels of tumor specificity during replication and safety, and is preferable for viral therapy.

Furthermore, in the present invention, there are no particular limitations on the methods for inhibiting expression of the above-mentioned genes (γ34.5 gene, ICP6 gene and ICP47 gene), and a suitable method can be selected by a person with ordinary skill in the art, examples of which include gene deletion methods and gene activation methods consisting of the insertion of other DNA at an intermediate location in the gene.

In the method for constructing recombinant HSV as claimed in the present invention, it is necessary to incorporate in advance a IoxP site and an FRT site in the HSV genome in order to utilize the Cre-IoxP and Flp-FRT systems, and the BAC system is used for this purpose. A BAC plasmid is an artificial infectant having a single copy of F factor plasmid of E. coli, and since it is able to maintain comparatively large DNA fragments in a stable state, it is useful for incorporating a desired exogenous gene into the genomic DNA of certain organisms. A BAC plasmid incorporated with a desired exogenous gene can be proliferated in E. coli, and if co-transfected into a host together with a linearized HSV genome (such as Vero cells or E. coli), can be incorporated into the HSV genome by homologous recombination. The procedure for incorporating a IoxP site and FRT site in a BAC plasmid, and co-transfection of the linearized BAC plasmid and HSV can be easily carried out by a person with ordinary skill in the art using known methods or methods complying thereto.

At least one type of marker gene is preferably inserted into the BAC plasmid to confirm whether or not the BAC plasmid has been incorporated in the HSV genome. A gene having a gene product which luminesces or which is resistant to a drug can be used as a marker gene.

Examples of genes which can be used as a marker gene by detecting luminescence of a gene product include GFP gene, RFP gene and luciferase gene. In addition, a gene resistant to an antibiotic is preferable for use as a drug-resistant gene, examples of which include a tetracycline-resistant gene, ampicillin-resistant gene, chloramphenicol-resistant gene, streptomycin-resistant gene, puromycin-resistant gene, kanamycin-resistant gene and neomycin-resistant gene. In addition, lacZ gene, which encodes β-galactosidase, and gusA gene, which encodes β-gtucuronidase, can also be used as marker genes. These genes are preferable since the products thereof, namely β-gatactosidase or β-glucuronidase, can be detected easily since the reactions which degrade their respective substrates are chromogenic reactions.

In the method for constructing recombinant HSV as claimed in the present invention, if these marker genes are inserted between the IoxP site and FRT site of a BAC plasmid, the marker gene is excised from the HSV genome by the Flp-FRT system in the third step. Thus, detection of the disappearance of these marker gene products can also be used to confirm whether or not the Flp-FRT system has functioned. In addition, since the marker gene is ultimately excised from the HSV genome, the marker protein may be harmful to the body of a human and so on.

On the basis of the above, although a marker gene which is inserted into the BAC plasmid and which facilitates selection of a target virus can be said to be useful regardless of whether or not it is harmful to humans, a gene which is expressed rapidly and encodes GFP demonstrating bright fluorescence is most preferable for the marker gene.

An expression cassette of a marker gene having a structure in which the marker gene is functionally linked downstream from a promoter is inserted into the BAC plasmid used in the present invention. In the present invention, "functionally linked" refers to a promoter and marker gene being bound so that binding by a transcription factor to a promoter results in initiation of transcription downstream there from.

A promoter sequence suitably selected from known promoters according to the type of marker gene desired to be expressed can be used for the promoter sequence used in the present invention, examples of which include cytomegalovirus (CMV) promoter, EF-1α promoter, β-actin promoter, SV40 promoter, TK promoter, P_{L} promoter, SRα promoter and RNA1.8 promoter.

Furthermore, insertion of IoxP site, FRT site and marker gene expression cassette into the BAC plasmid can be suitably carried out by a known method or method complying thereto, such as the method of Kim et al. (Kim S.Y et al.: Genome Res. 8: 404-12, 1998), the method of Kaname et at. (Kaname T., Huxley C: Gene 266: 147-53, 2001), or the method of Lee et al. (Lee E.C. et al.: Genomics 73: 56-65, 2001).

A "gene encoding a target protein" is inserted into a shuttle vector in the present invention. In the present invention, there are no particular limitations on the "gene encoding a target protein" provided it is a gene which encodes a protein exhibiting effects advantageous for treating or preventing cancer as a result of being expressed in cancer cells. Examples of such genes include immunostimulatory genes, genes encoding proteins having anti-neovascularizing action which effectively inhibit proliferation of cancer by inhibiting cancer-associated neovascularization, cancer inhibitory genes, and genes encoding cell membrane fusion proteins.

Examples of immunostimulatory genes include genes respectively encoding co-stimulatory factor containing secretory B7.1 (B7.1-Ig), interleukins such as IL-12, IL-18, IL-23 and IL-27, and transporter associated with antigen processing (TAP). Antitumor immunity is evoked and antitumor effects of viral therapy are enhanced as a result of these genes being expressed in cancer cells.

Examples of genes encoding proteins having anti-neovascularizing action include endostatin, angiostatin, dominant negative FGF receptor and platelet factor 4. Neovascularization can be inhibited in cancer cells and cancer proliferation can be inhibited as a result of these proteins being expressed in cancer cells.

Examples of tumor suppressor genes include genes such as p53, RB, WT1, NF1, NF2, DCC, APC, prohibitin, p16, BRCA1, MSH2, MLH1, PMS1, PMS2, VHL and IRF-1, and genes encoding fusion proteins containing these genes. Since tumor suppressor genes are inactivated are in a state in which cell proliferation is not inhibited in cancer cells, unlimited proliferation of cancer cells can be inhibited by inserting these genes into cancer cells.

Genes encoding virus surface proteins can be used as genes encoding cell membrane fusion proteins, and examples of such genes include genes encoding GALV, which is a cell fusion protein originating in gibbon leukemia virus, or its variant, GALV.fus (GALV-FMG) (X, Fu et al.: Molecular Therapy Vol. 7, No. 6, June 2003), genes encoding measles virus protein F and H (MV-F and MV-H) (Bateman A. et al.: Cancer Res 60: 1492-1497, 2000), genes encoding vesicular stomatitis virus G protein (rhabdovirus VSV-G envelope gene) (Elashi NK, Cancer Gene Ther 8: 55-62, 2001), genes encoding mutant F protein originating in Newcastle's disease virus (single amino acid substitution mutant (L289A) among a seven-time repeating motif (HR3) of NDV fusion protein), and genes encoding paramyxovirus SV5 cell membrane fusion protein (SV5F protein) (Gomez-Trevino A. et al.: J Gene Med 5: 483-492, 2003).

Cytocidal effects have been reported to be improved since cells infected with recombinant HSV rapidly fuse with surrounding cells as a result of inserting a gene encoding a cell membrane fusion protein into genomic DNA.

A gene encoding any of the above-mentioned target proteins is inserted into a shuttle vector in the form of an expression cassette having a structure in which the gene is functionally linked downstream from a promoter. A promoter which is not present in the genomic DNA of naturally-occurring HSV is preferably used for the promoter of the gene encoding the target protein. Although a promoter inserted into the above-mentioned BAC plasmid can be used for the promoter, CMV promoter is particularly preferable. As a result of using a promoter sequence which is not present in the naturally-occurring HSV genome, there is less susceptibility to the occurrence of unexpected mutations caused by homologous recombination, thereby making it possible to enhance the probability of obtaining the target recombinant HSV.

A loxP site and an FRT site are incorporated in the shuttle vector used in the present invention as previously described. As a result, the shuttle vector can be incorporated in the HSV genome using the Cre-loxP system, and unnecessary sequences can be excised from the HSV genome using the Flp-FRT system.

In addition, one or more types of marker genes are preferably inserted into the shuttle vector as claimed in the present invention to confirm that the shuttle vector has been incorporated in the HSV genome. Examples of marker genes include those having a luminescent gene product and drug-resistant genes.

There are no particular limitations on the marker gene, and that similar to marker genes inserted into the BAC plasmid as previously described can be suitably selected and used. Examples of marker genes which can be used include IacZ gene, gasA gene, GFP gene and RFP gene. Furthermore, the marker incorporated into the shuttle vector is preferably not a gene which encodes a protein which is harmful to humans since it remains in the final product without being excised from the HSV genome by the Flp-FRT system. An example of a marker gene which is not harmful to humans and fulfills the objective as a marker gene is the lacZ gene.

Furthermore, the marker gene inserted into the shuttle vector and the marker gene inserted into the BAC plasmid preferably have different properties. This makes it possible to clearly confirm whether or not the marker gene has been inserted into the shuttle vector.

These marker genes may be inserted into the shuttle vector in the form of a marker gene expression cassette containing a functionally linked promoter, only the marker gene may be inserted into the shuttle vector, and the marker gene may be expressed by using a promoter of the HSV genome.

In addition, the shuttle vector used in the method for constructing recombinant HSV as claimed in the present invention preferably has a stuffer sequence. A stuffer sequence refers to a DNA sequence which does not have any unnecessary action on virus growth. The naturally-occurring HSV genome is about 152 kb, and when the genome reaches a size of about 170 kb or larger, the properties no longer take on the form of a virus. In the third step of the method for constructing recombinant HSV as claimed in the present invention, unnecessary portions are excised from the HSV genome by the Flp-FRT system. Here, in the case the Flp-FRT system has not functioned properly as well, unless a stuffer sequence is present, the genome length is less than 170 kb, and a virus is formed which still contains unnecessary sequences in the genome thereof. However, when a stuffer sequence is inserted a virus is not formed since the genome length exceeds 170 kb in the case the Flp-FRT system has not functioned. Thus, the stuffer sequence inserted in the present invention preferably has a length of about 5000 nucleotides or more, more preferably a length of about 5500 nucleotides or more, even more preferably a length of about 5700 nucleotides or more, and most preferably a length of 5790 nucleotides or more. In the construction of a recombinant virus, since considerable effort is spent in the step for identifying those viruses in which recombination did not occur properly, as a result of inserting a stuffer gene, the present method is extremely useful since it enables viruses in which recombination has not occurred to be removed automatically.

An expression cassette of a gene encoding a target protein, a IoxP site, an FRT site, a marker gene expression cassette and a stuffer sequence can be inserted into the shuttle vector by a person with ordinary skill in the art using a known method or a method complying thereto.

As has been described above, the shuttle vector is incorporated into the HSV genome inserted with a BAC plasmid using the Cre-IoxP system, and a host is co-infected with the resulting HSV genome together with a vector able to express Flp recombinase. There are no particular limitations on the host cells provided they can be infected by HSV and can be cultured, and examples of cells which can be used include tumor cells and Vero cells. Preferable examples of cells include those originating in mammals such as humans or monkeys.

When Flp recombinase is expressed in Vero cells, the region surrounded by two FRT sites is excised from the HSV genome as a result of the Flp recombinase acting on both FRT sites in the HSV genome. As a result, the target recombinant HSV genome is obtained and HSV is produced. The final virus strain can be isolated by, for example, the limiting dilution method, and the viral DNA can be cleaved with a restrictase and confirmed by analyzing by Southern blotting.

In addition, the present invention also provides a pharmaceutical composition containing recombinant HSV constructed according to the method for constructing recombinant HSV as claimed in the present invention. A pharmaceutical composition as claimed in the present invention contains the recombinant HSV as an active ingredient thereof, and is useful for the treatment and prevention of various cancer diseases.

In the case of using a pharmaceutical composition as claimed in the present invention as a pharmaceutical, there are no particular limitations on the form of administration, and the pharmaceutical composition may be administered orally or parenterally. Although there are no particular limitations on the dose, in the case of administering to mammals (e.g., humans, mice, rats, guinea pigs, rabbits, dogs, horses or monkeys), and particularly to humans, although varying according to the degree of symptoms, age, gender and body weight of the patient, differences in sensitivity, administration method, administration period, properties, preparation and type of the pharmaceutical preparation, type of active ingredient and so on, the dose is about 10³ pfu to about 10¹² pfu, preferably about 10⁷ pfu to about 10¹⁰ pfu, and more preferably about 10⁸ pfu to about 5 × 10⁹ pfu, and the pharmaceutical composition can be administered by intravenous administration in a single administration of divided among multiple administrations.

A pharmaceutical composition as claimed in the present invention may use recombinant HSV as is, or it may be formulated according to commonly used methods by mixing with known pharmaceutically acceptable carriers, stabilizers, emulsifiers and so on. There are no particular limitations on the drug form, examples of which include capsules, syrups, inhalants, injection preparations, ointments, ophthalmic ointments, eye drops, nose drops, ear drops and lotions, although injection preparations are particularly preferable.

Examples of oral preparations include powders, grains, granules, tablets, enteric coated pills and capsules produced in accordance with ordinary methods following the addition of a vehicle, and as necessary, a binder, disintegration agent, lubricant, colorant or corrective and so on.

In the case of tablets and granules, the preparation may naturally be suitably coated as necessary with a sugar coating, gelatin coating or other coating.

In the case of injection preparations, the injection preparation may be freeze-dried, and can be administered into a tumor, vein, artery, under the skin, muscle, abdominal cavity or pleural cavity.

The above-mentioned pharmaceutical composition is useful for the prevention or treatment of various cancer diseases. Viral therapy using recombinant HSV is already known to be effective against all types of solid cancers (see, for example, the above-mentioned Todo, T et al: Tumor Suppressing Viruses, Genes, and Drugs - Innovative Cancer Therapy Approaches, San Diego, Academic Press: 45-75 (2001)), and the pharmaceutical composition as claimed in the present invention can be used for all of these cancers. Specific examples of such diseases include brain tumor, carcinoma of the head and neck, esophageal cancer, stomach cancer, colon cancer, liver cancer, pancreas cancer, lung cancer, breast cancer, skin cancer, ovarian cancer, prostate cancer, kidney cancer, urinary bladder cancer, melanoma and neuroblastoma.

Although the following provides an explanation of exemplary reference examples, examples and test examples of the present invention, the present invention is not limited to the specific examples indicated below. A person with ordinary skill in the art is able to maximally work the present invention by adding various modifications to the following examples, and those changes are included in the scope of the claims of the present application.

### Example 1

### (Construction of Recombinant HSV-1)

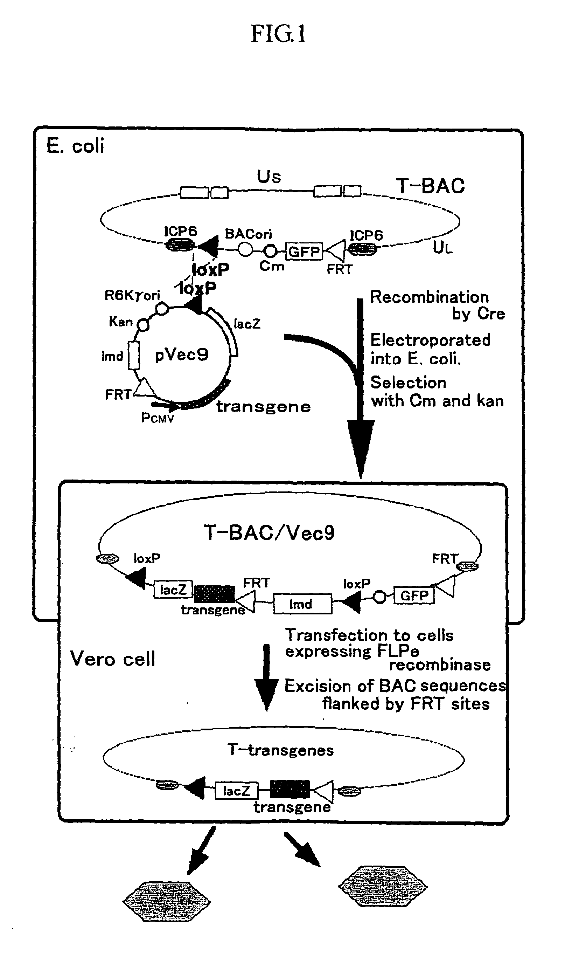

Fig. 1 shows a schematic representation of the method for constructing recombinant HSV as claimed in the present invention. In Fig. 1, a gene encoding a target protein (transgene) is represented. A schematic representation is shown in the upper portion of Fig. 1 of a step in which a shuttle vector, inserted with an expression cassette in which the transgene is functionally linked downstream from a CMV promoter, marker genes (IacZ and Kan), a IoxP site and an FRT site, is inserted into the IoxP site of a plasmid in which ICP6 gene and IacZ gene have deleted from a G47Δ genome, and inserted with a BAC sequence. The middle and lower portions of Fig. 1 schematically show a step in which an unnecessary region surrounded by FRT sites is excised from the construct prepared in this manner by Flp recombinase. The following provides a detailed explanation of each step.

In the present example, G47Δ virus genome is used for the skeleton of the recombinant HSV, while IL-18 and/or B7.1-Ig gene is used for the gene encoding the target protein.

### (Construction of BAC Plasmid)

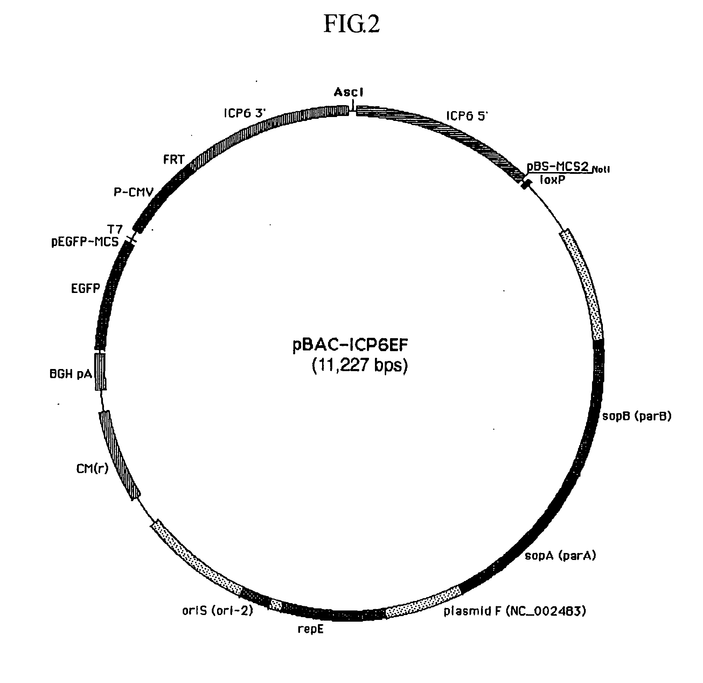

The following procedure was carried out on pBelobac11 (7507 base pairs) acquired from ResGen Corp. The pBelobac11 plasmid has a IoxP site and a marker gene in the form of a chloramphenicol (Cm)-resistant gene. The BAC plasmid (pBAC-ICP6EF) was constructed by inserting a sequence containing an FRT site, GFP gene and ICP6 gene of G47Δ DNA into the pBelobac11 plasmid. Fig. 2 shows the structure of pBAC-ICP6EF. A sequence having the 5'-terminal (approx. 1300 base pairs) and 3'-terminal (approx. 1200 base pairs) of ICP6 gene on both ends can be obtained by excising from the pBAC-ICP6EF plasmid with restrictase Asc1. A BAC plasmid replication site, Cm-resistant gene and GFP gene are arranged between the ICP6 genes so as to be surrounded by the IoxP site and FRP site.

### (Insertion of BAC Plasmid into G47Δ Genome)

E. coli was co-infected with G47Δ DNA and pBAC-ICP6EF, and the BAC plasmid was inserted at the ICP6 site of G47Δ DNA by homologous recombination. Since the pBAC-ICP6EF plasmid only has the 5'-terminal and 3'-terminal of the ICP6 site, G47Δ/BAC structure (upper section in Fig. 1, hereinafter called "T-BAC) is missing the approx. 1 kb center of the ICP6 site. G47Δ was 153 kb, while T-BAC was 157.7 kb. The viral plaque resulting from homologous recombination was observed with a fluorescence microscope, and T-BAC expressing GFP was selected. Continuing, electrocompetent E. coli DH10B was inserted to selectively proliferate Cm-resistant strains. The resulting T-BAC was purified and the structure thereof was confirmed using restrictase.

### (Construction of Shuttle Vector)

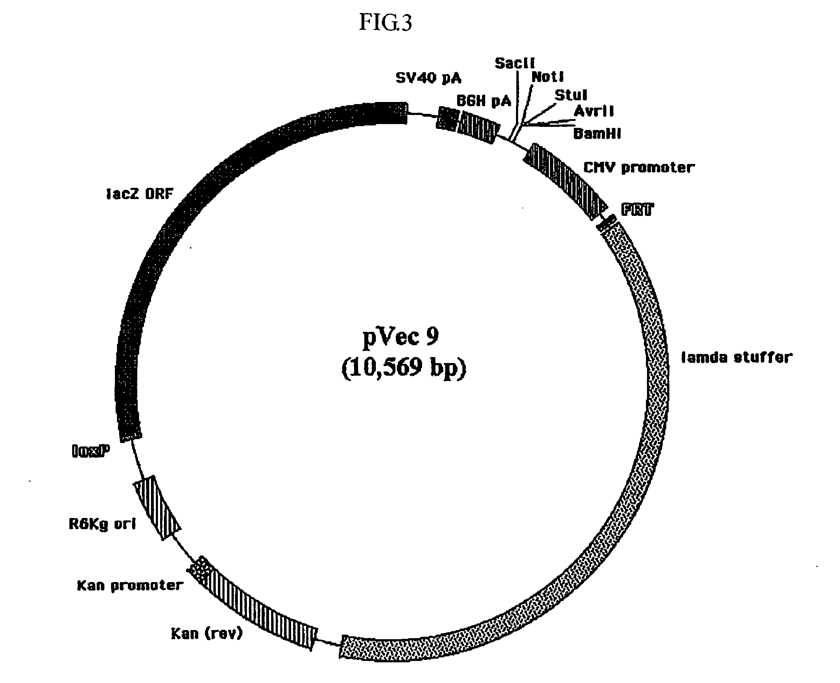

Three types of shuttle vectors were constructed according to the type of target protein to be expressed in cancer cells. The plasmid prior to insertion of gene encoding the target protein (pVec9: 10,569 bp) is shown in Fig. 3. Marker genes in the form of IacZ gene and kanamycin (Kan)-resistant gene were respectively incorporated so as to be able to be expressed, and a IoxP site, FRT site and stuffer region are also contained in the plasmid. A portion of a lambda sequence was used for the stuffer region. A gene encoding the target protein can be incorporated in the region recognized by restrictase located downstream from a CMV promoter in the upper right portion of the drawing. The region recognized by restrictase contains restrictase sites for BamHI, AvrII, StuI, NotI and SacII, and contrivances have been made to enable the incorporation of numerous genes.

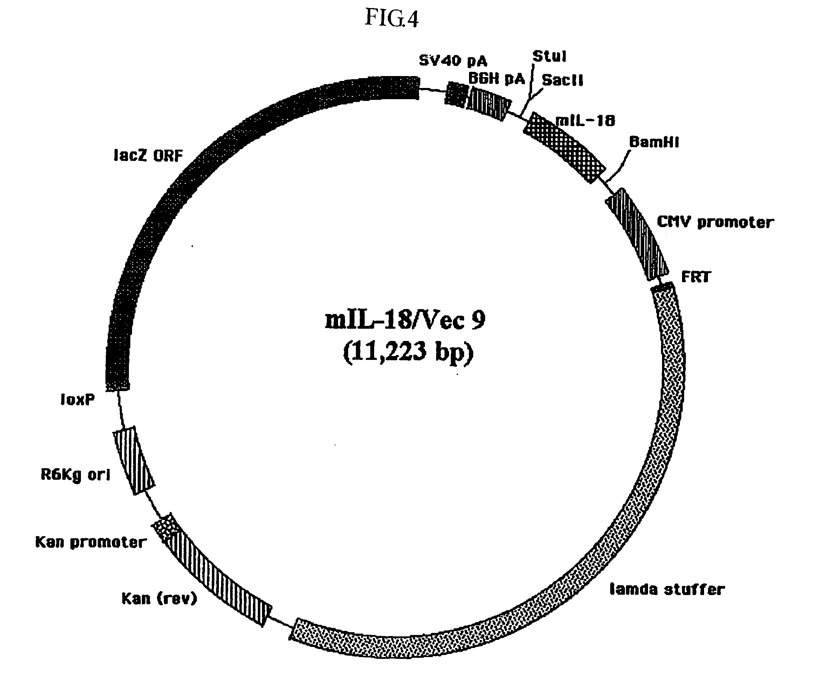

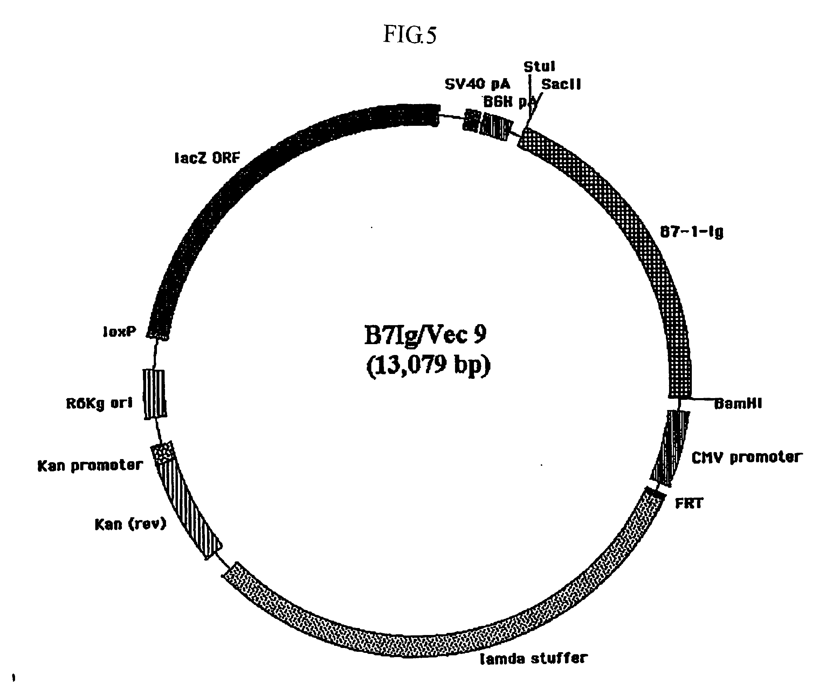

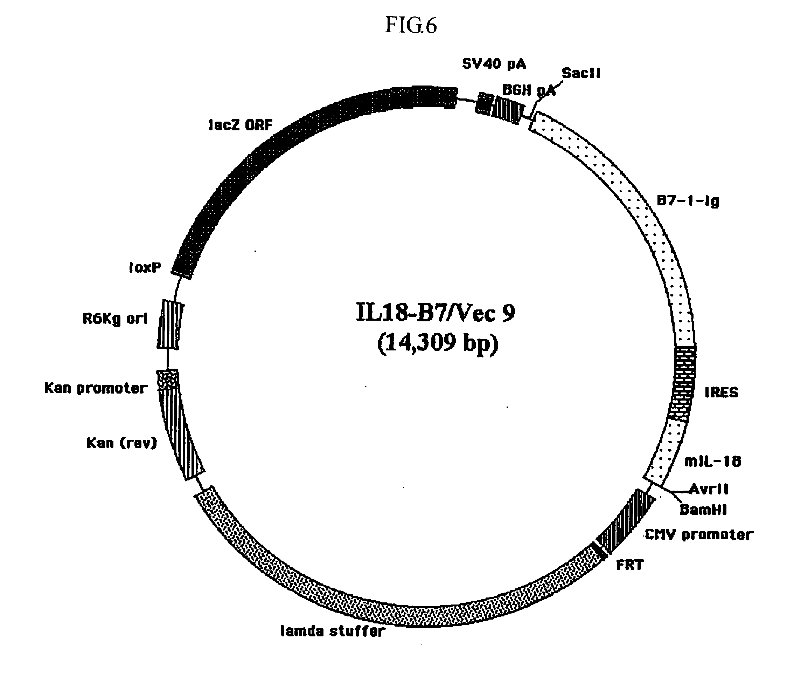

Fig. 4 shows the shuttle vector incorporated with a gene encoding a target protein in the form of mIL-18, Fig. 5 shows the shuttle vector incorporated with a gene encoding a target protein in the form of B7.1-Ig, and Fig. 6 shows the shuttle vector inserted with a gene encoding a fusion protein of mIL-18 and B7.1-lg. The internal ribosomal entry site (IRES) of encephalomyelitis virus (ECMV) was used to express multiple genes simultaneously.

### (Insertion of Shuttle Vector into T-BAC)

Continuing, the shuttle vector was inserted at the IoxP site of T-BAC using the Cre-loxP system (refer to the top and middle sections of Fig. 1). First, 150 ng of shuttle vector incorporating the target gene, 1500 ng of T-BAC and Cre recombinase acquired from New England Inc. were mixed in a 1.5 ml Eppendorf tube and allowed to stand for 30 minutes at 37°C. The reaction of this process only was completed followed by transformation of E. coli. According to this method, the need for the bothersome task of recombination within E. coli and the accompanying complex procedure were able to be omitted. This method of carrying out the reaction in solution is not only fast, but is also thought to contribute to reducing the risk of selecting an incorrect virus.

The target product was selected from the resulting transformants according to Kan resistance and Cm resistance on an LB plate. The target recombination was confirmed to have occurred in roughly 80% of the transformants.

### (Removal of Unnecessary Sequences)

Vero cells were co-infected with the T-BAC inserted with the shuttle vector together with a plasmid expressing FLPe (pCAGGSFIpIIRSpuro) followed by excising the region surrounded by the FRT sites (14.6 kb) (refer to the middle and lower sections of Fig. 1). FLPe is a site-specific recombinase developed in vitro originating in Saccharomyces cerevisiae.

As a result of infecting the Vero cells and observing the replicated viral plaque with a fluorescent microscope, plaque in which GFP was not expressed was selected as the target plaque. The BAC sequence was completely removed from the recombinant HSV-1 genome by excising the sequence using the Flp-FRT system. This means that the GFP gene having immunogenicity and toxicity is removed from the virus genome. The target virus not expressing GFP account for 99% or more of the virus recovered at this stage. Moreover, the target virus was obtained by carrying out purification twice using the limiting dilution, consisting of re-infecting Vero cells with the replicated virus and selecting virus plaque which does not express GFP. Whether or not this virus was the target virus was determined by confirming the sequence by Southern blotting using restrictase.

### (Virus Replication)

Two six-well plates in which 3 x 10⁵ cells were disseminated into each well were prepared, and one of the plates was infected with virus at an MOI of 0.01. Following infection, the plates were allowed to stand undisturbed for 48 hours at 37.0°C followed by collection of the cells and repeating freezing and thawing three times to obtain lysates. The titers of the viruses that increased in number in this manner were measured in Vero cells.

In addition, after disseminating 1 x 10⁶ cells into each well of a six-well plate, infecting with virus at an MOl of 1 and allowing to stand undisturbed for 24 hours, the cells were incubated for 48 hours at 39.5°C in the presence of ganciclovir (200 ng/ml) followed by ELISA assay and B7 staining. The kit of MBL Co., Ltd. was used for measurement of IL-18 by ELISA assay.

Those results are shown in Table 1.

**Table 1**

| | Replication Assay (pfu/ml) | IL-18 (pg/ml) | B7 staining |
|---|---|---|---|
| G47Δ | 2.0 x 10⁷ | | (-) |
| G47Δ-empty | 7.6 x 10⁶ | | (-) |
| G47Δ-IL18/B7-1 | 7.6 x 10⁶ | 1,002 | (+) |
| G47Δ-IL18/B7-2 | 4.4 x 10⁶ | 818 | (+) |
| G47Δ-IL18/B7-3 | 8.3 x 10⁶ | 986 | (+) |
| G47Δ-IL18/B7-4 | 5.7 x 10⁶ | 338 | (+) |
| G47ΔIL18-1 | 1.3 x 10⁷ | 1,418 | |
| G47Δ-IL18-2 | 3.2 x 10⁶ | 730 | |
| G47Δ-IL18-3 | 9.6 x 10⁶ | 1,710 | |
| G47Δ-IL18-4 | 7.6 x 10⁶ | 994 | |
| G47Δ-B7-1 | 1.6 x 10⁷ | | (+) |
| G47Δ-B7-2 | 3.9 x 10⁶ | | (+) |
| G47Δ-B7-3 | 1.2 x 10⁷ | | (+) |
| G47Δ-B7-4 | 5.8 x 10⁶ | | (+) |

G47Δ empty refers to a virus produced from the plasmid shown in Fig. 3 which does not contain a gene encoding the target protein.

Although the virus proliferation ability was somewhat inferior when compared with G47Δ, proliferation ability was confirmed to be adequate for use in viral therapy.

Viruses constructed by incorporating IL-18 and B7.1-Ig genes in the G47Δ genome were confirmed to simultaneously express both proteins, while viruses incorporating either of the genes were confirmed to express the respective protein.

### Example 2

### (Confirmation of Cytocidal Effects)

The cytocidal effects of G47Δ/IL-18, G47Δ/B7 and G47Δ/IL18/B7 against cancer cells in vitro were compared with G47Δ empty.

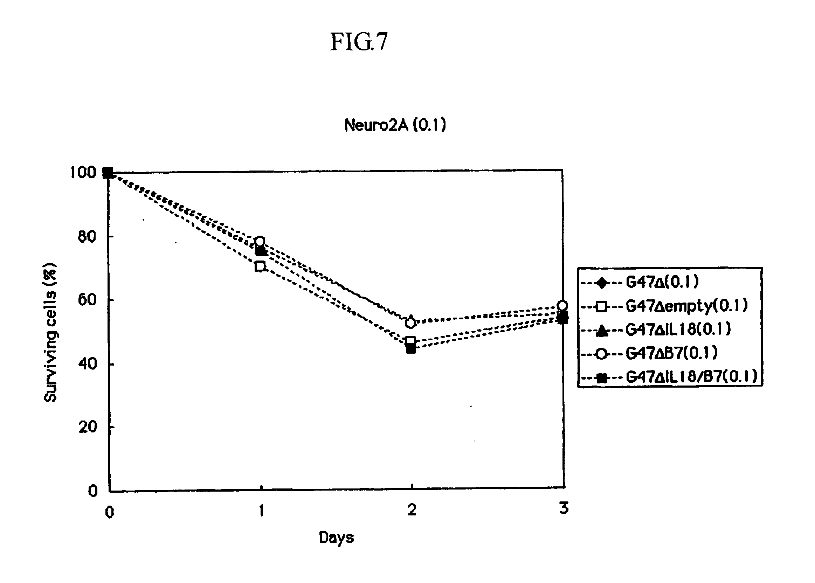

Fig. 7 shows the results of an experiment on a mouse malignant glioma cell line (Neuro2a). G47Δ/IL-18, G47Δ/B7 and G47Δ/IL18/B7 all demonstrated cytocidal effects at the same speed as G47Δ or G47Δ empty at low MOI (MOI = 0.1 ).

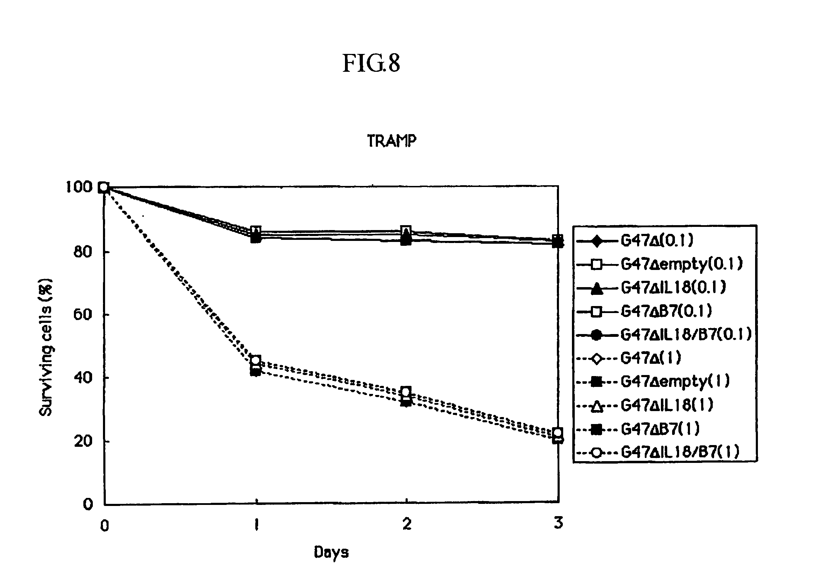

Fig. 8 shows the results of an experiment on a mouse prostate cancer cell line (TRAMP). G47Δ/IL-18, G47Δ/B7 and G47Δ/IL 18/B7 all demonstrated cytocidal effects at the same speed as G47Δ or G47Δ empty at low MOI (MOI = 0.1 and MOI = 0.01).

On the basis of the above, cytotoxicity comparable to G47Δ cells was confirmed to be maintained even if altered so as to express a target protein.

In addition, ELISA assays were carried out on IL-18 in Neuro2a, Pr14-2 (mouse prostate cancer cell line) and TRAMP. Those results are shown in Table 2.

**Table 2**

| | mIL-18 (pg/ml) |
|---|---|
| G47Δ-IL18/B7 in Neuro2a | 538 |
| G47Δ-IL18/B7 in Pr14-2 | 572 |
| G47Δ-IL18/B7 in TRAMP | 806 |
| G47Δ-IL18 in Neuro2a | 483 |
| G47Δ-IL18 in Pr14-2 | 658 |
| G47Δ-IL18 in TRAMP | 673 |

| | |
|---|---|
| All of the viruses were confirmed to express IL-18 at 500 to 800 pg/ml. | |

### Example 3

### (Confirmation of Antitumor Effects)

Antitumor effects were tested against a mouse tumor model using the viruses described above.

5×10⁶ TRAMP-C2 mouse prostate cancer cells were subcutaneously injected into mice of the syngeneic strain (C57Bl/6, male). Seven days later, the mice were randomly assigned to groups and designating the day of grouping as day 0, the mice underwent therapy using each of the viruses. Each group of mice was injected with 5x10⁶ pfu/20 µl of G47Δ, G47Δ empty, G47Δ/IL18, G47Δ/B7 and G47Δ/IL18/B7 on days 0 and 3 into the tumor tissue.

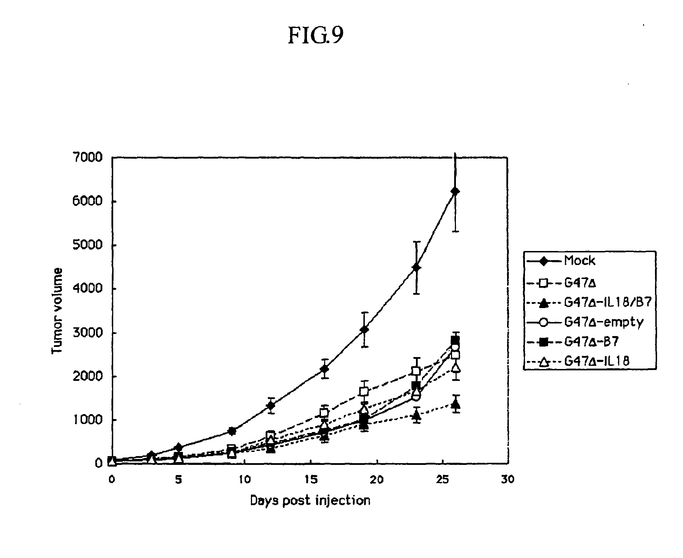

Those results are shown in Fig. 9. In the group administered G47Δ/IL18/B7, the volume of the TRAMP-C2 tumor decreased remarkably. The presence of a statistically significant difference was tested with the t-test. The group administered G47Δ/IL18/87 was concluded to demonstrate a remarkable decrease in TRAMP-C2 tumor volume on day 19 as compared with the group administered G47Δ/IL18 at a level of significance of 5%. Similarly, the group administered G47Δ/IL18/B7 was concluded to demonstrate a remarkable decrease in TRAMP-C2 tumor volume on day 21 as compared with the G47Δ/B7 group at a level of significance of 5%.

5 x 10⁷ Neuro2a cells (100 µL) were injected into both sides of A/J mice. Similar to the TRAMP-C2 model, 5 x 10⁵ pfu/20 µl of G47Δ, G47Δ empty, G47Δ/IL18, G47Δ/B7 and G47Δ/IL18/B7 were injected into the tumor tissue. Antitumor effects were similar to those observed with TRAMP-C2.

### Example 4

### (Expression of IL-12)

1 x 10⁶ Vero cells were disseminated in each well of a 24-well plate and cultured overnight. Three clones each of a virus in which the ICP6 gene of G47Δ was missing prepared using T-BAC (T-empty), and a virus in which mouse IL-12 gene was inserted at the ICP6 gene site of G47Δ (T-mfIL12), were infected for 1 hour at an MOI of 2 in two wells each. After culturing for 18 hours at 37°C and 39.5°C, the content of mouse IL-12 was measured by ELISA assay. Although there was no expression of IL-12 observed at all in the case of infection by T-empty, potent expression of IL-12 was observed in the case of infection by T-mfIL12. A large amount of expression was observed at 37°C, at which virus replication is particularly active, as compared with that at 39.5°C. Those results are shown in Table 3.

**Table 3**

| | 37°C | 39.5°C |
|---|---|---|
| #1-1 | 19.2 | 5.0 |
| #1-2 | 19.0 | 4.4 |
| #2-1 | 24.0 | 4.2 |
| #2-2 | 21.4 | 4.3 |
| #3-1 | 24.0 | 6.6 |
| #3-2 | 26.2 | 6.2 |

### Example 5

### (Antitumor Effects of T-mfIL12 in a Mouse Subcutaneous Tumor Model)

A virus in which the ICP6 gene of G47Δ was missing (T-empty), a virus inserted with mouse IL-12 gene at the site of the ICP6 gene of G47Δ (T-mfIL12), and a control in the form of PBS containing 10% glycerol (mock) were injected twice on days 0 and 3 into the tumor tissue on the left side only using A/J mice having a Neuro2a subcutaneous tumor measuring about 5 mm in diameter on both flanks (5-6 mice per virus). The dimensions of the tumor tissue were measured, and the volume was determined from the length x width x height (mm).

The results are shown the figure. With respect to the tumor on the left side, increases in tumor volume were significantly inhibited in the T-empty dose group (■) and T-mfIL 12 dose group (▲) as compared with the mock dose group (◆). Moreover, the T-mfIL 12 dose group demonstrated significantly more potent antitumor effects than the T-empty dose group. In addition, with respect to the distant tumor on the right side, although significant inhibitory effects on increases in tumor volume were not indicated in the T-empty dose group as compared with the mock dose group, the T-mfIL12 dose group demonstrated significant inhibition of increases in tumor volume as compared with the mock dose group and T-empty dose group.

On the basis of these results, recombinant herpes simplex virus incorporating IL-12 gene was confirmed to enhance antitumor effects as compared with recombinant herpes simplex virus not incorporating a transgene.

## Claims

1. A method for constructing recombinant herpes simplex virus capable of expressing a target protein in cancer cells, comprising the steps of:
inserting into a herpes simplex virus genome, a BAC plasmid, which has a IoxP site and an FRT site and into which has been inserted at least one type of marker gene expression cassette having a structure in which a marker gene is functionally linked downstream of a promoter, between the IoxP site and the FRP site;
constructing a shuttle vector into which has been respectively inserted at least one type of expression cassette of a gene encoding the target protein having a structure in which the gene encoding the target protein is functionally linked downstream of a promoter, at least one type of marker gene, a IoxP site and an FRP site, and inserting said shuttle vector into the IoxP site of the herpes simplex virus genome using Cre recombinase so as to realize a constitution which allows expression of the gene encoding the target protein and the marker gene; and,
co-infecting a host with the herpes simplex virus genome obtained in the second step and a vector capable of expressing Flp recombinase, and excising the region between the FRT sites in said genome to produce a target recombinant herpes simplex virus.

2. The method according to claim 1, wherein the second step is carried out in a liquid phase.

3. The method according to claim 1 or claim 2, wherein a γ34.5 gene and ICP6 gene of the herpes simplex virus are deleted or inactivated prior to the first step.

4. The method according to claim 3, wherein ICP47 gene of the herpes simplex virus is additionally deleted or inactivated.

5. The method according to any of claims 1 to 4, wherein the marker gene inserted into the BAC plasmid is a gene encoding green fluorescent protein (GFP) and/or an antibiotic resistance gene.

6. The method according to any of claims 1 to 5, wherein the promoter contained in at least one type of expression cassette of a gene encoding the target protein is a promoter comprising a nucleotide sequence not present in the naturally-occurring herpes simplex virus genome.

7. The method according to any of claims 1 to 6, wherein the promoter comprising a nucleotide sequence not present in the herpes simplex virus genome is CMV promoter.

8. The method according to any of claims 1 to 7, wherein the marker gene inserted into the shuttle vector is IacZ gene and/or an antibiotic resistance gene.

9. The method according to claim 8, wherein the marker gene inserted into the shuttle vector is an antibiotic resistance gene different from the antibiotic resistance gene inserted into the BAC plasmid.

10. The method according to any of claims 1 to 9, wherein the gene encoding the target protein is one or more genes selected from the group consisting of an immunostimulatory gene, a anti-angiogenesis gene, a gene encoding a cell membrane fusion protein, and a tumor suppressor gene.

11. The method according to claim 10, wherein the immunostimulatory gene is a gene encoding one or more proteins selected from the group consisting of co-stimulatory factor, IL-12, IL-18, IL-23, IL-27 and transporter associated with antigen processing (TAP).

12. The method according to claim 10, wherein the anti-angiogenesis gene is a gene encoding one or more proteins selected from the group consisting of endostatin, angiostatin, dominant negative FGF receptor and platelet factor 4.

13. The method according to claim 10, wherein the gene encoding a cell membrane fusion protein is a virus surface protein.

14. The method according to claim 10, wherein the tumor suppressor gene is p53 gene.

15. The method according to any of claims 1 to 14, wherein the shuttle vector contains a stuffer sequence.

16. The method according to claim 15, wherein the stuffer sequence is about 5000 nucleotides or more in length.

17. A recombinant herpes simplex virus constructed according to the method in any one of claims 1 to 16.

18. A pharmaceutical composition containing a recombinant herpes simplex virus according to claim 17.

19. The pharmaceutical composition according to claim 18, which is a therapeutic or preventive of various cancer diseases.

20. A method for preventing or treating cancer, comprising administration of the pharmaceutical composition according to claim 18.
